Europäisches Patentamt

(19) European Patent Office

Office européen des brevets

(11) Numéro de publication: **0 053 976**
**B1**

(12) **FASCICULE DE BREVET EUROPEEN**

(45) Date de publication du fascicule du brevet: **17.10.90**

(21) Numéro de dépôt: **81401917.0**

(22) Date de dépôt: **03.12.81**

(51) Int. Cl.⁵: **G 01 L 9/00, G 01 L 7/10, A 61 B 5/02**

(54) Dispositif pour capter et exploiter une pression notamment de fluide.

(30) Priorité: **05.12.80 FR 8025815**
**25.06.81 FR 8112457**

(43) Date de publication de la demande:
**16.06.82 Bulletin 82/24**

(45) Mention de la délivrance du brevet:
**17.10.90 Bulletin 90/42**

(84) Etats contractants désignés:
**AT BE CH DE GB IT LI LU NL SE**

(56) Documents cités:
**FR-A- 771 345**
**FR-A-1 291 996**
**FR-A-2 439 988**
**US-A-3 535 067**
**US-A-3 704 708**

(73) Titulaire: **Cousin, Bernard Maurice Jean Robert**
**F-37240 Montresor (Indre et Loire) (FR)**

(72) Inventeur: **Cousin, Bernard Maurice Jean Robert**
**F-37240 Montresor (Indre et Loire) (FR)**

(74) Mandataire: **Rataboul, Michel**
**Cabinet Michel Rataboul 69, rue de Richelieu**
**F-75002 Paris (FR)**

Courier Press, Leamington Spa, England.

**Description**

Il existe de très nombreux cas dans lesquels il est nécessaire de capter une pression, notamment de fluide, et de l'exploiter soit à titre de mesure, soit à titre de signal transcodable.

On peut citer, par exemple, la production de sons à partir des gravures d'un disque qui fait appel à la pression variable que les gravures font subir à une pointe de lecture, la mesure de la pression atmosphérique pour la construction de baromètres ou d'altimètres, la pression d'un corps placé sur le plateau d'une balance pour en mesurer le poids, la pression sanguine dans les artères d'un corps vivant pour en déduire des informations médicales, etc.

Parmi ces innombrables cas, il en est un qui est particulièrement remarquable et qui est celui de la pression artérielle. En effet, s'agissant d'une opération effectuée sur le corps humain, on doit tenir compte non seulement des données physiologiques mais également des facteurs psychologiques tout particulièrement à l'égard de la douleur éventuelle que cette mesure pourrait occasionner.

Jusqu'à maintenant, le moyen le plus répandu consiste à obturer complètement l'artère à étudier, en utilisant un brassard gonflable, ou garrot. Il est clair qu'un tel moyen ne permet pas de mesurer la pression sanguine ailleurs que sur un membre.

On a donc pensé à utiliser d'autres moyens de captation, comme celui qui est décrit dans le document US-A-3,704,708. Le dispositif décrit dans ce document est destiné à capter la pression sanguine et pour l'exploiter à titre de mesure afin d'en dériver un signal transcodable. Il comprend d'une part une capsule qui contient un liquide et qui est munie d'une ouverture obturée par une membrane souple accessible à la pression devant être captée et susceptible d'occuper diverses positions et, d'autre part, une partie située à l'opposé de l'ouverture et pourvue à son extrémité de moyens élastiquement déformables sous l'action de ladite pression.

Mais ce document met en oeuvre des moyens différents de ceux de l'invention.

En effet, le document US-A-3,704,708 est basé sur le principe que le fonctionnement du dispositif ne dépend pas des propriétés du système vasculaire mais seulement de la transmission fidèle d'une pression hydrostatique (colonne 4, lignes 33 à 37 et colonne 7, lignes 5 à 10).

Le fonctionnement du dispositif suppose que l'on aplatit le vaisseau étudié (colonne 4, lignes 40 et 41) et nécessite la présence d'un os de squelette (colonne 3, ligne 44 et 45).

C'est pourquoi le tube capillaire 110 est muni d'un bouchon amovible 124 (colonne 6, ligne 48) par lequel il peut être temporairement obturé (colonne 6, lignes 66 et 67).

En opération, le bouchon 124 doit être retiré (colonne 7, lignes 16 à 20) puis remis en place.

Ainsi, on replace le bouchon, 124 après montée du liquide 126 (qui est soumis à la pression atmosphérique), ce qui signifie qu'il n'y a pas de contre-pression étalonnée. Si on replace le bouchon 124 avant montée du liquide, la contre-pression fournie par l'air emprisonné a une valeur inconnue et qui varie à chaque mesure puisque le volume d'air est variable (le liquide 126 est plus ou moins haut quand on remet le bouchon 124).

On connaît aussi le brevet US-A-3,535,067 qui décrit un dispositif pour capter la pression sanguine et pour l'exploiter à titre de mesure. Il comprend d'une part une capsule qui contient un liquide et qui est munie d'une ouverture obturée par un clapet rigide à ressort, cette ouverture étant soit fermée, soit ouverte, auquel cas elle laisse libre le passage du liquide vers un vérin relié à un circuit hydraulique comprenant un conduit aller partant d'une chambre solidaire de la capsule et un conduit retour aboutissant à la capsule.

Le clapet n'est pas déformable et, lorsqu'il est en appui sur son siège, il n'exerce aucune action sur le liquide de la capsule.

Il existe une contre-pression au niveau du vérin mais cette contre-pression n'a qu'un retour indirect et retardé à la capsule recevant la pression étudiée, par une fuite et un circuit hydraulique.

Cela s'oppose à l'invention avec laquelle la contre-pression s'exerce directement et rapidement dans la capsule recevant la pression étudiée, ce qui permet d'observer la variation dans le temps de la pression artérielle, avec ses aspects caractéristiques. Cela permet de calculer l'élasticité du vaisseau étudié, la vitesse de propagation de l'onde de pression sanguine et la pression artérielle dans des zones aussi critiques que le centre de la rétine ou la carotide et, cela, grâce au fait que l'on ne garotte pas le vaisseau étudié mais que l'on ne fait qu'appliquer le dispositif sur un pouls quelconque.

La présente invention apporte donc une solution nouvelle aux prolèmes de la mesure d'une pression et utilise des moyens simples et très efficaces.

A cette fin, l'invention a pour objet un dispositif pour capter une pression, notamment de fluide, et pour l'exploiter à titre de mesure afin d'en dériver un signal transcodable, du type comprenant d'une part une capsule qui contient au moins un liquide et qui est munie d'une ouverture obturée par une membrane souple accessible à la pression devant être captée et susceptible d'occuper diverses positions, et d'autre part une partie située à l'opposé de l'ouverture et pourvue à son extrémité de moyens élastiquement déformables sous l'action de ladite pression, caractérisé en ce que lesdits moyens élastiquement déformables comportent au moins une membrane placée de manière étanche sur une deuxième ouverture, et en ce que ces moyens exercent une contre-pression sur le liquide pour chaque valeur de la pression captée et sont aptes à se déformer de manière rapide et étalonnée afin de transmettre une information représentative de cette pression.

Selon des modes de réalisation de l'invention:
le dispositif comporte un détecteur constitué

par un ensemble cellule photo-électrique — projecteur, dont l'axe lumineux est transversal à un écran mobile relié cinématiquement à la membrane élastique, cette membrane obturant ladite seconde ouverture cette dernière étant plus petite que la précédente, la capsule étant entièrement remplie du liquide;

l'écran est constitué par une extrémité éventuellement coudée d'une lame élastique faisant partie des moyens élastiquement déformables et assujettie par son autre extrémité à une partie fixe et munie d'un pion appliqué contre la face extérieure de la seconde membrane par la lame élastique;

le dispositif comprend un corps qui présente extérieurement la première membrane souple et qui est adapté à être maintenu plus ou moins longtemps à un endroit voulu de la surface d'un corps vivant pour que cette membrane soit soumise à la pression résultant de l'augmentation volumique radiale d'un segment artériel dans lequel se développe une pression sanguine;

le corps est associé à des organes de maintien rigides tels qu'un bracelet inélastique;

les moyens élastiquement déformables sont constitués par un élément de transcodage des informations mécaniques en informations exploitables telles qu'électriques, électroniques et analogues;

la capsule contient une troisième membrane déformable, transversale, fixée par sa périphérie et qui détermine deux chambres isolées remplies de liquide, dont l'une comporte la première ouverture et l'autre comporte les moyens élastiquement déformables;

la chambre comportant les moyens élastiquement déformables a une paroi courbe ayant une forme équivalente à celle que peut prendre la troisième membrane transversale lorsqu'elle subit une déformation maximum afin que cette membrane transversale prenne alors appui sur la paroi courbe qui lui sert de butée;

la chambre comportant les moyens élastiquement déformables a une paroi courbe en forme de calotte sphérique;

le dispositif comprend des moyens pour lui appliquer une contre-pression résultant d'une mesure recueillie en un point différent de celui auquel le dispositif est appliqué par sa première membrane souple, la mesure finalement obtenue résultant de la somme algébrique de la pression et de la contre-pression;

le dispositif comprend un boîtier à deux entrées dont l'une correspond à la deuxième ouverture de la capsule dont la membrane est constituée par un détecteur piézoélectrique et extérieurement le liquide de la capsule tandis que l'autre reçoit un tube dont l'extrémité libre est fermée par une membrane, le liquide remplissant le tube et le boîtier pour être en contact du détecteur piézoélectrique mais par sa face opposée à celle qui reçoit le liquide de la capsule afin d'obtenir une mesure différentielle des pressions;

le boîtier est traversé d'une ouverture obturée par une membrane souple;

l'extrémité de la capsule constitue le sommet d'un col et reçoit la deuxième membrane associée à un embout portant un écran susceptible d'être situé en regard d'orifices transversaux pour un ensemble détecteur optique, la membrane et l'embout étant de même section, laquelle est égale, au jeu près, à celle de l'intérieur du col;

l'extrémité de la capsule constitue le sommet d'un col et est percé d'un trou par lequel s'étend un axe d'un piston portant un écran extérieur à la capsule, la deuxième membrane étant percée en son centre et étant interposée entre le fond de la capsule et le piston.

L'invention sera mieux comprise par la description détaillée ci-après faite en référence au dessin annexé. Bien entendu, la description et le dessin ne sont donnés qu'à titre d'exemple indicatif et non limitatif.

Les figures 1 et 2 sont deux vues schématiques partielles en coupe d'un dispositif conforme à l'invention selon un premier mode de réalisation.

La figure 3 est une vue schématique montrant un exemple de réalisation d'un appareil complet comprenant le dispositif conforme à l'invention.

La figure 4 est un schéma montrant l'association d'un dispositif conforme à l'invention avec des appareils permettant l'exploitation de l'information donnée par un dispositif conforme à l'invention.

Les figures 5 et 6 sont deux vues schématiques en coupe d'un dispositif conforme à l'invention selon un deuxième mode de réalisation et dans deux positions correspondant respectivement à la captation d'une pression nulle et d'une pression positive.

La figure 7 est une vue schématique en coupe d'un autre mode de réalisation de l'invention.

La figure 8 est une vue schématique en coupe d'un autre mode de réalisation de l'invention.

La figure 9 est une vue schématique en coupe d'un dispositif conforme à l'invention et du type comprenant une plaquette piézoélectrique associée aux moyens élastiquement déformables.

Les figures 10 et 11 illustrent schématiquement l'utilisation du dispositif de la figure 9.

Les figures 12 et 13 sont deux vues schématiques en coupe de deux variantes d'un autre mode de réalisation de l'invention.

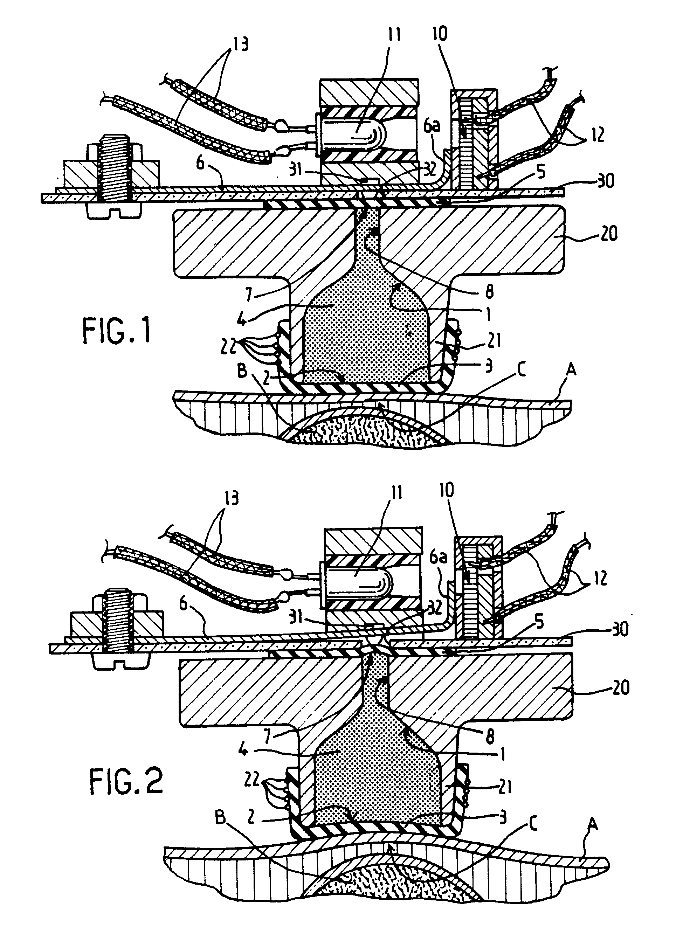

En se reportant aux figures 1 et 2, on voit qu'un dispositif conforme à l'invention est distiné à capter une pression notamment de fluide et à l'exploiter soit à titre de mesure, soit à titre de signal transcodable et est du type comprenant une capsule 1 qui est munie d'une ouverture 2 obturée par une membrane souple 3 accessible à la pression devant être captée et qui contient au moins un liquide 4. Ce dispositif comprend des moyens élastiquement comprimables situés à l'opposé de l'ouverture 2 et exerçant une contre-pression étalonnée et invariable sur le liquide 4 afin d'assurer le rappel automatique et instantané de la membrane souple 3 obturant l'ouverture 2. Ces moyens sont constitués par une membrane 5 éventuellement associée à un ressort 6. A l'opposé de l'ouverture 2 se trouve une autre ouverture 7 qui est plus petite que la précédente 2.

En outre, la partie de la capsule 1 opposée à l'ouverture 2 est située à l'extrémité d'un col 8.

On voit ainsi qu'en soumettant la membrane 3 à la pression à mesurer, et en maintenant la capsule 1 pour que celle-ci reste immobile, la pression déforme la membrane 3 en faisant varier le volume interne de la capsule 1 dans le sens d'une diminution, ce qui a pour conséquence de faire monter le liquide 4 dans le col 8.

Le volume déplacé dans la zone large de la capsule 1 reste obligatoirement le même que celui qui se déplace dans le col 8, mais celui-ci étant beaucoup plus étroit, il est clair que la hauteur sur laquelle se produit le mouvement de liquide 4 est beaucoup plus importante que celle que subit, dans la même direction, la membrane 3.

Il en résulte qu'un très petit déplacement de la membrane 3 se traduit par un déplacement beaucoup plus sensible du liquide 4 à l'intérieur du col 8, ce qui permet d'effectuer une mesure très précise en exploitant le déplacement du niveau du liquide 4.

Afin de pouvoir détecter non seulement une pression donnée mais les variations instantanées de cette pression, il est indispensable que le liquide 4 soit soumis à une contre-pression étalonnée assurant le rappel automatique et instantané de la membrane 3.

On peut réaliser pratiquement un dispositif conforme à l'invention en plaçant une capsule indépendante remplie et fermée dans une ouverture d'un support puis en coiffant l'ensemble au moyen d'une pièce que l'on fixe.

Sur les figures 1 et 2, on a représenté un mode de réalisation de l'invention selon lequel le détecteur est constitué par un ensemble cellule photo électrique 10 projecteur 11 dont l'axe lumineux est transversal à celui de la membrane 3 et alimenté par des fils électriques 12 et 13.

Le fonctionnement de ce détecteur est bien connu en lui-même et l'on sait qu'en alimentant l'ampoule du projecteur 11 par un courant électrique conduit par les fils 13, ce projecteur 11 émet un faisceau lumineux qui atteint la cellule photo électrique 10 qui envoie par les fils 12 un courant électrique proportionnel à l'éclairement qu'elle reçoit du faisceau émis par le projecteur 11.

Tant que le liquide opaque et 4 est à la pression d'étalonnage zéro, le courant électrique issu de la cellule 10 est exploité dans un appareil de tout type connu éventuellement associé à un écran de lecture de telle sorte que cette situation corresponde à l'indication d'une absence de pression. Lorsque le faisceau est interrompu, le courant créé par la cellule 10 a une valeur différente et l'appareil indique cette différence qui peut être soit la simple indication de l'existence d'une pression, soit une indication précise de cette pression en établissant une relation entre la valeur de celle-ci et la fraction de faisceau lumineux occulté.

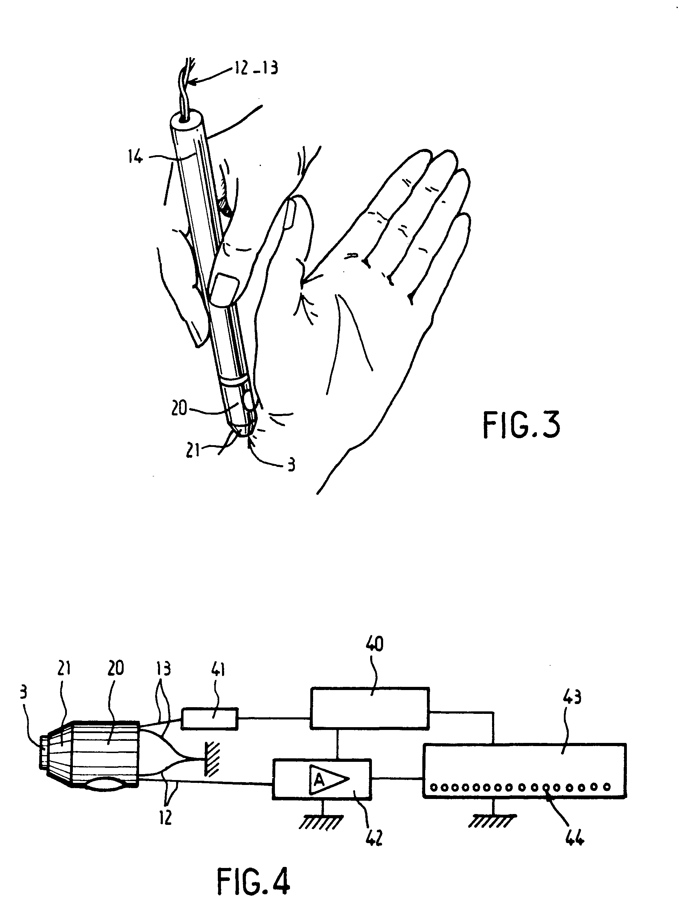

On a choisi d'illustrer l'invention par un dispositif adapté à la mesure de la tension artérielle et l'on voit sur la figure 3 que le dispositif proprement dit tel que représenté sur les figures 1 et 2, peut être associé à un corps 14 que le praticien peut manipuler comme un crayon.

L'invention est, en effet, particulièrement intéressante pour cette application car les appareils à brassard gonflable ne permettent pas de prendre la tension artérielle dans des artères telles que la carotide, l'aorte abdominale, l'artère centrale de la rétine et beaucoup d'autres car elles se trouvent dans des zones où il est impossible de placer le garrot que constitue le brassard gonflable.

En outre, la mesure de la tension artérielle ne peut pas être effectuée en continu et suppose l'intervention du praticien.

Il n'est donc pas possible avec les dispositifs connus de mesurer l'élasticité artérielle ni de mesurer la vitesse de propagation de l'onde de pression sanguine, alors que cela est obtenu avec un dispositif conforme à l'invention.

Naturellement, on connaît le procédé qui consiste à agir par voie sanglante mais cela n'est possible qu'à l'occasion d'une intervention chirurgicale et, de toutes façons, ce procédé ne permet pas l'étude de toutes les artères pouls par pouls.

Le dispositif conforme à l'invention, au contraire, du fait de la transduction des informations purement mécaniques de la membrane 3 en informations électriques permet ensuite l'exploitation de ce signal électrique au moyen d'appareils de toutes sortes, connus en euxmêmes.

On peut, ainsi, obtenir un graphique ou un affichage permanent sur un écran ou une mise en mémoire ou une exploitation informatique, soit indépendante, soit elle-même associée à des dispositifs complémentaires tels que stimulateurs cardiaques, systèmes d'alarmes etc.

On voit en effet, que grâce à l'invention on peut mesurer la pression artérielle en appliquant la membrane 3 sur un pouls quelconque y compris dans une zone non garrotable.

Le dispositif indique les pressions exercées sur la membrane 3 placée au contact de la peau A alors que le déplacement de la membrane 3 est inférieur au déplacement de la même surface de la paroi de l'artère B lorsque celle-ci n'est pas soumise au dispositif, moins le déplacement absorbé par le tissu intermédiaire C soumis à ces pressions.

L'augmentation volumique du segment artériel B lors du passage de l'onde de pression sanguine doit recevoir l'action antagoniste de la contre-pression créée dans le dispositif et non pas la réaction de la paroi de l'artère B.

Pour que ces conditions soient convenablement remplies, on peut soit tenir le dispositif comme représenté sur la figure 3 c'est-à-dire pendant un temps court, soit utiliser des organes de maintien rigides tels qu'un bracelet inélastique.

En associant le dispositif non plus à un corps 14 en forme de crayon mais à une embase peu élevée, il est possible de fixer le dispositif à l'endroit voulu pendant des temps relativement longs et, par exemple, pendant quelques jours ou quelques nuits de surveillance intensive justifiée par un état grave du patient.

On peut aussi placer plusieurs dispositifs aux endroits les plus représentatifs, par exemple lors d'une intervention chirurgicale ou à l'occasion d'examens poussés.

On voit ici que la capsule est créée par une cavité convenablement conformée à l'intérieur d'une pièce 20 qui présente un embout 21 sur lequel la membrane 3 est maintenue au moyen de liens 22 qui ceinturent l'ensemble.

La cavité, ou capsule, est entièrement remplie d'un liquide incompressible 4 et la petite ouverture 7 est obturée par la membrane élastique 5.

On voit que l'ensemble cellule photo-électrique 10-projecteur 11 a son axe lumineux transversal à un écran mobile relié cinématiquement à la membrane élastique 5 qui obture la petite ouverture 7 de la capsule 1.

Selon une variante, l'écran est constitué par une extrémité coudée 6a de la lame élastique 6 assujettie par son autre extrémité à une partie fixe 30 et munie d'un pion 31 appliqué contre la face extérieure de la membrane 5 par la lame élastique 6.

La partie fixe 30 est reliée par tout moyen connu à la pièce 20 et est percée d'un trou 32 dans lequel le pion 31 passe librement.

Avec ces dispositions, on voit que de petits déplacements de la membrane 3 provoquent des déplacements plus importants de la membrane 5, laquelle repousse le pion 31 et, par conséquent, la lame 6 et l'écran 6a qui vient se placer plus ou moins complètement en regard du faisceau lumineux pour l'occulter au moins en partie, d'où il résulte un courant variable dans les fils 13.

Lorsque la membrane 3 subit une pression inférieure ou nulle, le liquide incompressible 4 et la membrane élastique 5 reprennent leur position d'origine sous l'effet du pion 31 qui est poussé par la lame élastique 6, cet ensemble créant la contre-pression voulue à une valeur connue.

Le dispositif est complètement stabilisé à la chaleur puisque la lame élastique 6 est pratiquement insensible aux écarts de température.

Lorsqu'on utilise un détecteur optique à cellule photo-électrique, on peut supprimer le projecteur 11, ce qui diminue la consommation de courant électrique en utilisant la lumière ambiante. On place, alors, en bascule deux cellules photo-électriques qui reçoivent toutes les deux la lumière ambiante mais l'une est associée à un écran du type 6a de sorte que finalement celle qui reçoit la lumière ambiante directe sans être influencée par le dispositif, sert de référence à celle qui est associée à l'écran.

Pour mesurer le déplacement d'une paroi artérielle en vue de calculer un indice d'élasticité artérielle, un dispositif selon l'invention doit présenter une contre-pression inférieure à la contre-pression exercée par la paroi artérielle. Sur la figure 1, le déplacement de la membrane 3 est alors égal au déplacement de la même surface de la paroi de l'artère B moins le déplacement absorbé par le tissu intermédiaire C. L'appareil indique alors un déplacement amplifié. Le rapport entre ce déplacement d'une surface et la variation de pression sur cette même surface donne l'indice d'élasticité de l'artère.

Sur la figure 4 on a représenté un exemple d'association d'un dispositif portatif conforme à l'invention et d'appareils fixes.

Sur cette figure, on voit que l'ensemble de l'installation comprend une alimentation 40 en courant électrique de préférence à bassd tension telle que 12 Volts, un redresseur 41 à l'entrée du projecteur, un amplificateur 42 à la sortie de la cellule photoélectrique et, enfin, un dispositif d'affichage 43 comprenant ici une série de voyants lumineux 44 dont l'allumage et l'extinction sont contrôlés par les signaux amplifiés de la cellule photo-électrique, lesquels sont créés par les mouvements de la membrane 3.

Il ressort de la description ci-dessus, qu'un dispositif conforme à l'invention permet de transformer en signaux électriques des impulsions mécaniques nées d'une pression dont la valeur varie plus ou moins vite.

L'invention trouve donc un nombre très grand d'applications et, selon le cas, on peut aisément faire varier les deux composantes essentielles qui sont le déplacement volumique d'une part et la contre-pression d'autre part.

On peut, par exemple, appliquer l'invention à la fabrication de baromètres, d'altimètres ou de tachymètres (selon le principe du tube de Pitot en utilisant la contre-pression latérale d'écoulement du fluide) et tout système de jauge de liquides.

On peut utiliser un plateau intermédiaire rigide pour réaliser une balance.

En associant une masse mobile à la membrane 3, on peut mesurer l'accélération ou la décélération subie par le dispositif.

Les matériaux dont on dispose actuellement permettent de réduire au maximum l'inertie du liquide incompressible de sorte que l'on peut appliquer l'invention à la fabrication de têtes de lecture pour des tourne-disques, ou à la fabrication de micros, étant souligné que l'on peut accorder la surface externe du dispositif à la longueur d'ondes à étudier.

On peut également appliquer l'invention selon le principe de la balance à la surveillance et à l'entretien de plantes disposées dans un bac: quand l'humidité de la masse de terre baisse, la pression exercée sur la membrane du dispositif fixée sous le bac diminue et l'on peut prévoir qu'à partir d'un certain seuil, le dispositif commande l'ouverture d'une électro-vanne qui alimente le bac en eau d'arrosage.

On peut également étudier la propagation d'une onde de pression dans une soufflerie en plaçant en ligne une série de dispositifs et en enregistrant, simultanément, la valeur donnée par chacun des dispositifs de sorte que l'on obtient, à la fois, les pressions et la vitesse de propagation de l'onde.

Selon un mode de réalisation particulier, les moyens élastiquement comprimables sont associés à un élément de transcodage des informations mécaniques transmises par ledit organe en informations exploitables telles qu'électriques, électroniques et analogues.

Par exemple, le mode de réalisation représenté sur les figures 1 et 2 peut comprendre non plus un ensemble optique et un écran mobile mais une plaquette piézorésistante (non représentée) appliquée contre le membrane 5 afin de transformer en courant électrique les pressions absolues ou différentielles transmises à la plaquette par la membrane 5. Tout autre élément de transcodage sensible aux pressions et/ou aux mouvements de l'organe élastiquement comprimable peut être utilisé selon les cas et ce choix est à la portée de l'homme de métier (variations de capacités, variations de champs magnétiques, etc.).

Lorsque le dispositif est appliqué à la mesure de la pression sanguine, on peut rechercher une miniaturisation suffisante pour l'implanter dans le corps. On peut, notamment, le placer dans la région cardiaque, contre une côte, qui l'immobilise.

Dans ce cas, on doit retenir les solutions les plus aptes à diminuer l'encombrement du dispositif et les contraintes qu'il pourrait imposer. On peut, à titre d'exemple, utiliser un ensemble interne comprenant d'une part le "capteur" proprement dit constitué de la capsule 1 et de sa membrane 3 et d'autre part deux fibres optiques aboutissant à un appareil de lecture externe (projecteur et cellule photo-électrique).

En vue de supprimer les fils électriques de liaison, on peut prévoir un émetteur radio miniature implanté avec le capteur et un récepteur extérieur associé à tout appareil voulu: afficheur, alarme, informatique etc.

Selon un mode de réalisation qui vient d'être décrit, les moyens élastiquement comprimables sont constitués par une membrane élastique qui doit se déformer d'une valeur relativement importante puisque la conception même de ce dispositif prévoit que le liquide incompressible se déplace sur une hauteur plus grande en regard de cette membrane qu'à l'entrée de la capsule, là ou est placée une autre membrane soumise directement à la pression à mesurer.

Dans la pratique, il peut être difficile de trouver une matière qui remplisse à la fois la fonction de contre-pression, la fonction d'étanchéité et la fonction de grand déplacement.

On se heurte, en effet, à des exigences inconciliables d'élasticité et de résistance etc.

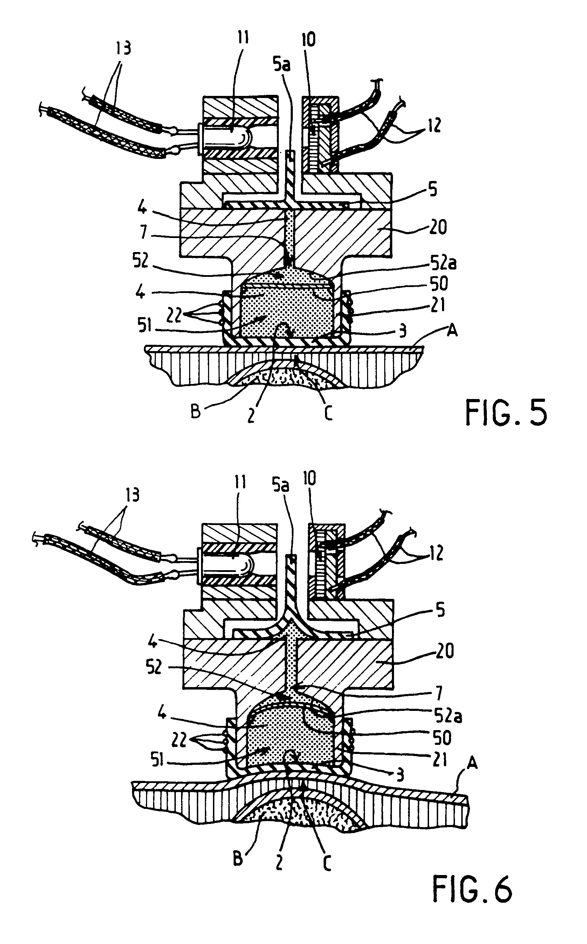

Grâce aux modes de réalisation qui vont maintenant être décrits en regard des figures 5 à 13 on résout ce problème de manière efficace.

En se reportant aux figures 5 et 6, on voit un dispositif conforme à l'invention réalisé selon le mode de réalisation prévoyant qu'un corps 20 présente un embout 21 sur lequel la membrane 3 est maintenue au moyen de liens 22 qui ceinturent l'ensemble.

Ici la capsule (ou la cavité prévue à l'intérieur de la pièce 20) contient une membrane déformable transversale 50 fixée par sa périphérie et qui détermine deux chambres isolées 51 et 52 remplies d'un liquide 4, l'une 51, comportant l'ouverture la plus grande 2 et l'autre 52, comportant l'ouverture la plus petite 7.

Avec ces dispositions, on voit, en se reportant particulièrement à la figure 6, que la déformation de la membrane 3 provoque la déformation de la membrane transversale 50 et c'est donc celle-ci qui assure pour l'essentiel la fonction de contre-pression.

En revanche, le déplacement du liquide incompressible 4 dans la chambre 52 a pour effet le soulèvement de la membrane 5 et ce soulèvement peut être exploité par les différents moyens exposés plus haut.

Grâce à ces dispositions, la membrane 5 peut être réalisée dans un matériau tel qu'elle assure aussi précisément que possible la fonction d'étanchéité et la fonction de soulèvement, indépendamment de la fonction de contre-pression.

En d'autres termes, on dissocie la fonction de contre-pression (ou "étalonnage") et la fonction étanchéité-déplacement.

La première fonction est assurée essentiellement par la membrane 50 qui peut être métallique, en matière synthétique etc. tandis que la deuxième fonction est assurée par la membrane 5.

On note que l'on peut alors choisir la matière de la membrane 50 indépendamment du rapport:

$$\frac{\text{diamètre}}{\text{souplesse}}$$

Ainsi, on peut avoir une membrane 50 en métal de grand diamètre pour avoir une flèche correcte lors de la déformation due à la mesure de pression et une membrane 5 petite et très souple.

Pour constituer la membrane 50, on peut, par exemple, utiliser une feuille de clinquant de six centièmes d'épaisseur pour un diamètre de six millimètres, ce qui procure exactement la flèche voulue pour déplacer dans la chambre 52 le volume de liquide incompressible 4 suffisant.

On note que sur les figures 5 et 6 on a choisi comme mode de réalisation de la mesure de pression la combinaison d'une cellule photoélectrique 10 et d'un projecteur 11 entre lesquels se déplace un écran transversal 5a obtenu en une seule pièce lors de la fabrication de la membrane 5.

Le fonctionnement de ce dispositif est en tous points identique à celui qui a été décrit plus haut.

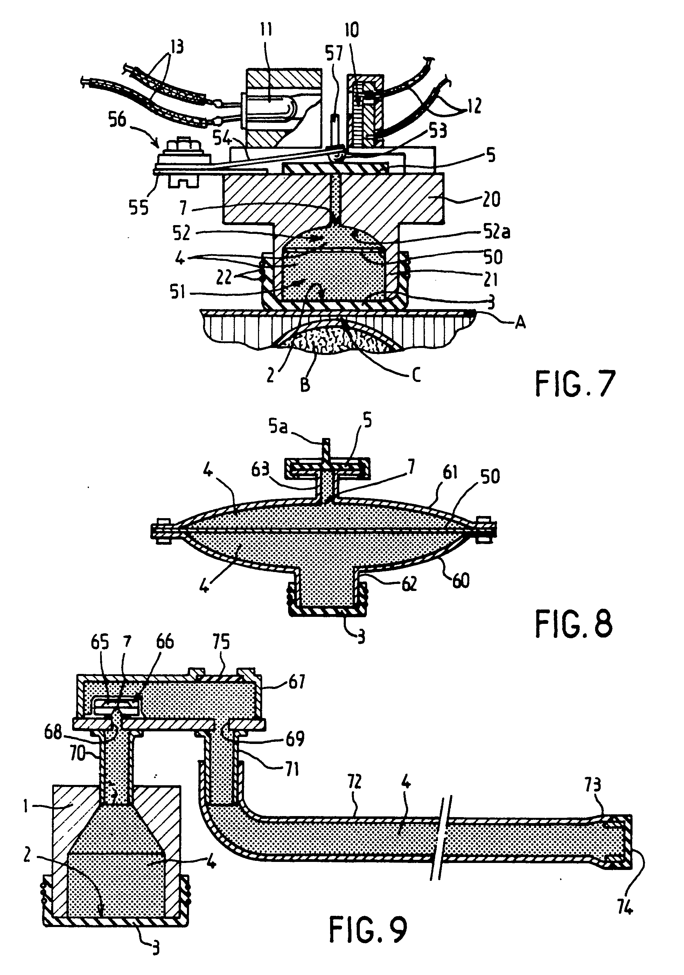

En se reportant maintenant à la figure 7, on voit un autre mode de réalisation selon lequel la membrane 5 est plate c'est-à-dire qu'elle ne comporte pas l'écran 5a. Elle reçoit en son centre un pion 53 rappelé par une lame de ressort 54 maintenue contre une partie fixe 55 au moyen d'un ensemble vis-écrou 56 et sur laquelle est fixé un écran 57.

Lorsque la membrane 5 est soulevée par le déplacement du liquide incompressible 4 situé dans la chambre supérieure 52, l'écran 57 vient s'interposer entre le pinceau lumineux du projecteur 11 et la cellule photo-électrique 10 comme cela a été décrit précédemment et lorsque le liquide incompressible 4 rentre de nouveau dans la chambre 52, la membrane 5 reprend sa place primitive de par son élasticité propre tandis que l'écran 57 et le pion 53 sont ramenés par retour élastique de la lame 54.

Selon un mode de réalisation de l'invention, la chambre 52 comportant l'ouverture la plus petite 7 a une paroi courbe 52a ayant une forme équivalente à celle que peut prendre la membrane transversale 50 lorsqu'elle subit une déformation maximum, afin que cette membrane 50 prenne alors appui sur la paroi courbe 52a qui lui sert de butée.

Selon une variante, la paroi courbe 52a de la chambre 52 a la forme d'une calotte sphérique.

Ces dispositions sont particulièrement visibles sur la figure 6 où l'on a représenté la membrane 50 déformée par l'effet d'une pression appliquée contre la membrane 3.

On voit ainsi que la membrane 50 se trouve assez près de la paroi 52a et présente un rayon de courbure un peu inférieur.

Cela signifie que la pression exercée sur la membrane 3 est inférieure à un maximum préétabli et calculé en fonction de la capacité de déformation de la membrane 50.

Si, au contraire, la membrane 3 était soumise (accidentellement ou pas) à une pression excessive par rapport à l'élasticité de la membrane 50, celle-ci serait brusquement déformée mais serait appliquée contre la paroi 52a et serait donc retenue au lieu d'être brisée ou dissociée de l'épaulement de la pièce 10 contre lequel elle est normalement assujettie.

Ces dispositions constituent donc une sécurité qui protège le dispositif contre une utilisation anormale ou un accident, ce qui pourrait se produire si, en laissant échapper le dispositif, celui-ci tombe sur le sol et rencontre ce dernier par la membrane 3.

On voit que l'invention permet aussi de choisir le diamètre de la membrane 50 en n'étant soumis qu'à un seul paramètre qui est la valeur de la flèche à obtenir.

Sur la figure 8 on a représenté un dispositif conforme à l'invention dont les proportions sont volontairement exagérées mais qui montrent l'indépendance complète qu'il y a entre le diamètre de la membrane 3, le diamètre de la membrane 50 et le diamètre de la membrane 5.

Le dispositif est, par ailleurs, obtenu par assemblage de deux demi-coquilles 60 et 61 présentant chacune un col respectivement 62 et 63 de diamètres différents.

La coquille supérieure 61 a une forme en calotte sphérique pour servir de butée à la membrane 50 ainsi que cela a été expliqué plus haut.

Avec un tel dispositif, on peut obtenir une amplification considérable du déplacement linéaire entre son origine, au niveau de la membrane 3 et son point d'application, au niveau de la membrane 5.

Un tel mode de réalisation, comprenant une membrane 50 de diamètre très grand par rapport au diamètre des autres membranes, est intéressant lorsque l'on doit choisir pour constituer cette membrane 50 un matériau relativement peu élastique et ne pouvant supporter qu'une flèche très faible ce qui implique, pour compenser cette faiblesse, de disposer d'un grand diamètre pour que la calotte sphérique créée par la déformation de la membrane 50 soit d'un volume suffisant pour donner un déplacement linéaire significatif au niveau de la membrane 5.

En se reportant maintenant à la figure 9, on voit un mode de réalisation de l'invention selon lequel on utilise une plaquette piézoélectrique de manière telle que l'on puisse capter une pression, par exemple au poignet, et en effectuer une mesure précise, quelles que soient les différences de niveau entre le poignet et le coeur.

On a représenté le cas où la capsule 1 ne contient pas de membrane intermédiaire 50 mais, bien entendu, elle pourrait aussi en contenir une, conformément aux indications données plus haut.

Plus précisément, on voit sur la figure 9 que l'ouverture 7 de plus petit diamètre communique avec une cavité 65 prévue dans un ensemble 66 de type connu et constituant une piézorésistance.

L'ensemble 66 est placé dans un boîtier 67 qui comprend deux entrées 68 et 69 prolongées par des embouts 70 et 71.

La capsule 1 est raccordée à l'embout 70 et l'on a prévu suffisamment de liquide incompressible 4 pour que soit rempli tout l'espace délimité par l'intérieur de la capsule 1, de l'embout 70, de l'entrée 68 et de la cavité 65.

Sur l'embout 71 est placé un tube flexible 72 dont l'extrémité 73 est obturée par une membrane 74. Un liquide incompressible 4 est placé dans tout l'espace délimité par le tube 72 l'embout 71 et l'intérieur du boîtier 67.

De la sorte, la piézorésistance 66 reçoit sur l'une de ses faces la pression captée par la membrane 3 et sur l'autre de ses faces, une pression éventuelle régnant dans le boîtier 67 selon la position de l'extrémité 73 du tube 72.

Lorsque l'on utilise le dispositif, on place la membrane 3 sur l'endroit voulu (généralement le poignet) et l'on place l'extrémité 73 à la même hauteur que le coeur.

Dans le cas de la figure 9, la membrane 3 est à la même hauteur que le coeur. Le capteur (non représenté) associé à la piézorésistance 66 donne alors une mesure qui correspond à la réalité, le tube 72 n'intervenant que de manière virtuelle.

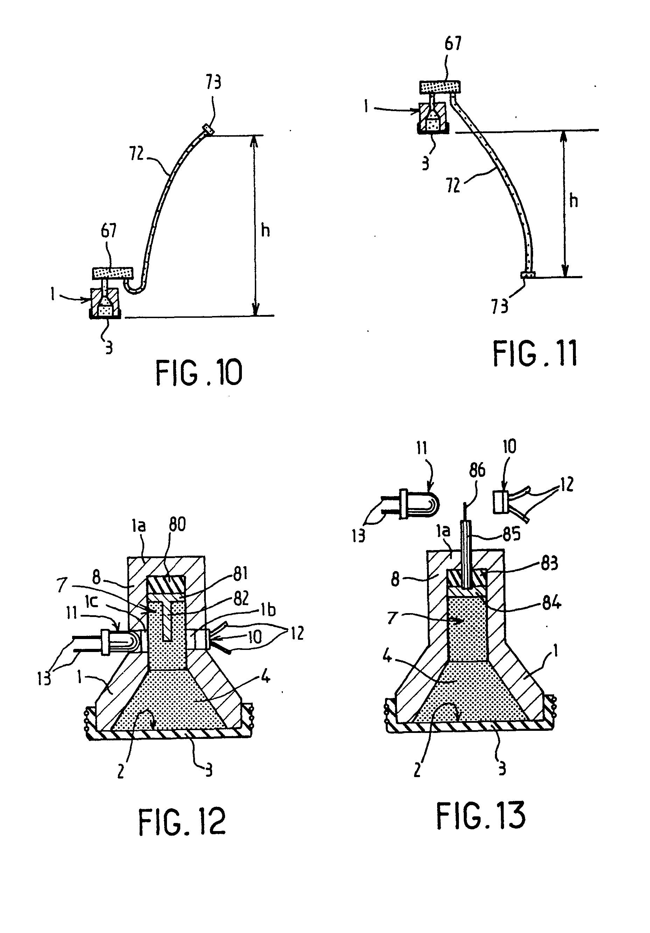

Dans le cas de la figure 10, la membrane 3 est à un niveau inférieur à celui du coeur, d'une hauteur h. Le capteur indique la pression sur la membrane 3, moins la pression de la colonne liquide contenue dans le tube 72 et qui est fonction de la hauteur h.

La piézorésistance 66, en effet, donne la valeur de la somme algébrique de ces deux pressions puisqu'elles sont appliquées sur les deux faces opposées de ladite piézorésistance 66.

Dans le cas de la figure 11, le capteur indique la pression sur la membrane 3, plus la pression de la colonne liquide car, ici, la membrane 3 est située plus haut que le coeur (hauteur $h$).

On remarque sur le boîtier 67 (figure 9) une ouverture obturée par une membrane élastique 75. Cela a pour but de permettre les déplacements de la piézorésistance 66 sans rencontrer la résistance qu'opposerait la masse de tout le liquide 4 contenu dans le boîtier 67 et le tube 72, ce liquide 4 ne devant que compenser la pression hydrostatique naturelle. La membrane 75 a donc pour effet de modérer le "travail" du pouls en diminuant la masse de liquide 4 en mouvement.

Par ailleurs, il existe des élastomères dont les caractéristiques physiques sont plus constantes dans le temps si elles sont utilisées en pression plutôt qu'en extension.

On peut donc, pour avoir une contre-pression constante, utiliser des membranes faites en ces matériaux et travaillant non pas en extension, comme décrit, mais en pression.

On sait que les matériaux modernes ont des performances excellentes permettant d'assurer pendant plusieurs années le fonctionnement de ces membranes placées dans ces conditions de fonctionnement favorables.

Cela est notamment le cas sur les figures 12 et 13.

Sur la figure 12, on a représenté un dispositif du type selon lequel la détection du mouvement résultant de la déformation de la membrane 3 s'opère au niveau de la capsule 1 et non au-dessus d'elle. Le fonctionnement de l'ensemble cellule photo-électrique 10 — projecteur 11 suppose, par conséquent, que la capsule 1 soit transparente au moins en face de la cellule 10 et du projecteur 11.

On a choisi, ici, de représenter un mode de réalisation selon lequel la capsule 1 ne comprend qu'une seule ouverture 2.

A l'opposé de celle-ci, la capsule 1 est fermée et contre le fond 1$a$ est placée une membrane élastique 80 qui reçoit un embout 81 de même surface et portant un écran 82. Les déplacements de l'écran 82, dus à l'enfoncement de la membrane 80, sont analysés à travers le liquide incompressible 4, transmetteur de pression, qui doit être transparent. La cellule 10 et le projecteur 11 sont placés dans des orifices latéraux 1$b$ et 1$c$ de la capsule 1.

Sur la figure 13, on a représenté une variante qui combine les dispositions précédentes pour ce qui est de la membrane travaillant à la compression et celles des figures 1-2 et 5-6 pour ce qui est du mode de détection.

La capsule 1 n'a pas d'orifices 1$b$ et 1$c$ et le liquide 4 peut être opaque. Une membrane élastique 83 est comprimée par un petit piston plat 84 de même surface, sur lequel est monté un axe 85 qui porte un écran 86. La membrane 83 est, en fait, un petit joint torique comprimé par la pression exercée sur le piston 84 par le liquide incompressible 4.

Les déplacements de l'écran 86 sont analysés avec une cellule photo-électrique 10 éclairée par une lampe 11.

L'ensemble de détection optique étant au-dessus de la capsule 1, celle-ci est traversée d'un trou par lequel passe l'axe 85 portant l'écran 86.

Grâce à l'invention, on capte l'énergie dynamique du vaisseau étudié, par simple contact sur la peau, sans appui important et, surtout, sans obturer le vaisseau étudié et sans le confiner.

Pour mesurer la pression, il fait que la contre-pression puisse s'exercer et que sa valeur soit connue. C'est pourquoi le volume de liquide incompressible 4 déplacé dans la capsule 1 pour une variation de pression donnée, doit être inférieur au volume disponible depuis la surface, à l'aplomb du vaisseau que l'on étudie.

Pour l'application de l'invention à la mesure d'une pression sanguine, il existe donc une limite physique absolue: le déplacement du liquide incompressible ne peut qu'être inférieur à celui d'une artère.

## Revendications

1. Dispositif pour capter une pression, notamment de fluide, et pour l'exploiter à titre de mesure afin d'en dériver un signal transcodable, du type comprenant d'une part une capsule (1) qui contient au moins un liquide (4) et qui est munie d'une ouverture (2) obturée par une membrane souple (3) accessible à la pression devant être captée et susceptible d'occuper diverses positions, et d'autre part une partie située à l'opposé de l'ouverture (2) et pourvue à son extrémité de moyens élastiquement déformables sous l'action de ladite pression, caractérisé en ce que lesdits moyens élastiquement déformables (5, 6, 80, 83) comportent au moins une membrane (5, 66, 80, 83) placée de manière étanche sur une deuxième ouverture (7), et en ce que ces moyens (5, 6, 80, 83) exercent une contre-pression sur le liquide (4) pour chaque valeur de la pression captée et sont aptes à se déformer de manière rapide et étalonnée afin de transmettre une information représentative de cette pression.

2. Dispositif selon la revendication 1, caractérisé en ce qu'il comporte un détecteur constitué par un ensemble cellule photoélectrique (10) — projecteur (11) dont l'axe lumineux est transversal à un écran mobile (5a, 6a) relié cinématiquement à la membrane élastique (5), cette membrane (5) obturant ladite seconde ouverture (7) cette dernière étant plus petite que la précédente (2), la capsule (1) étant entièrement remplie du liquide (4).

3. Dispositif selon la revendication 2, caractérisé en ce que l'écran est constitué par une extrémité (6a) éventuellement coudée d'une lame élastique (6) faisant partie des moyens élastiquement déformables et assujettie par son autre extrémité à une partie fixe (30) et munie d'un pion (31) appliqué contre la face extérieure de la seconde membrane (5) par la lame élastique (6).

4. Dispositif selon la revendication 1, caractérisé en ce qu'il comprend un corps (14, 20, 21) qui présente extérieurement la première membrane souple (3) et qui est adapté à être maintenu plus

ou moins longtemps à un endroit voulu de la surface d'un corps vivant pour que cette membrane (3) soit soumise à la pression résultant de l'augmentation volumique radiale d'un segment artériel dans lequel se développe une pression sanguine.

5. Dispositif selon la revendication 4, caractérisé en ce que le corps (20, 21) est associé à des organes de maintien rigides tels qu'un bracelet inélastique.

6. Dispositif selon la revendication 1, caractérisé en ce que les moyens élastiquement déformables sont constitués par un élément de transcodage (66) des informations mécaniques en informations exploitables telles qu'électriques, électroniques et analogues.

7. Dispositif selon la revendication 1, caractérisé en ce que la capsule (1) contient une troisième membrane (50) déformable, transversale, fixée par sa périphérie et qui détermine deux chambres isolées (51 et 52) remplies de liquide (4), dont l'une (51) comporte la première ouverture (2) et l'autre (52) comporte les moyens élastiquement déformables.

8. Dispositif selon la revendication 7, caractérisé en ce que la chambre (52) comportant les moyens élastiquement déformables a une paroi courbe (52a) ayant une forme équivalente à celle que peut prendre la troisième membrane transversale (50) lorsqu'elle subit une déformation maximum afin que cette membrane transversale (50) prenne alors appui sur la paroi courbe (52a) qui lui sert de butée.

9. Dispositif selon la revendication 8, caractérisé en ce que la chambre (52) comportant les moyens élastiquement déformables a une paroi courbe (52a) en forme de calotte sphérique.

10. Dispositif selon la revendication 1, caractérisé en ce qu'il comprend des moyens (66-72) pour lui appliquer une contre-pression résultant d'une mesure recueillie en un point différent de celui auquel le dispositif est appliqué par sa première membrane souple (3), la mesure finalement obtenue résultant de la somme algébrique de la pression et de la contre-pression.

11. Dispositif selon la revendication 10, caractérisé en ce qu'il comprend un boîtier (67) à deux entrées (68 et 69) dont l'une (68) correspond à la deuxième ouverture de la capsule (1) dont la membrane est constituée par un détecteur piézoélectrique (66) et extérieurement le liquide (4) de la capsule (1) tandis que l'autre (69) reçoit un tube (72) dont l'extrémité libre (73) est fermée par une membrane (74), le liquide (4) remplissant le tube (72) et le boîtier (67) pour être en contact du détecteur piézoélectrique (66) mais par sa face opposée à celle qui reçoit le liquide (4) de la capsule (1) afin d'obtenir une mesure différentielle des pressions.

12. Dispositif selon la revendication 11, caractérisé en ce que le boîtier (67) est traversé d'une ouverture obturée par une membrane souple (75).

13. Dispositif selon la revendication 1, caractérisé en ce que l'extrémité (1a) de la capsule (1) constitue le sommet d'un col (8) et reçoit la deuxième membrane (80) associée à un embout (81) portant un écran (82) susceptible d'être situé en regard d'orifices transversaux (1b et 1c) pour un ensemble détecteur optique (10-11), la membrane (80) et l'embout (81) étant de même section, laquelle est égale, au jeu près, à celle de l'intérieur du col (8).

14. Dispositif selon la revendication 1, caractérisé en ce que l'extrémité (1a) de la capsule (1) constitue le sommet d'un col (8) et est percé d'un trou par lequel s'étend un axe (85) d'un piston (84) portant un écran (86) extérieur à la capsule (1), la deuxième membrane (83) étant percée en son centre et étant interposée entre le fond (1a) de la capsule (1) et le piston (84).

**Patentansprüche**

1. Vorrichtung zum Erfassen eines Druckes, insbesondere von Flüssigkeiten, und zur genauen Auswertung der Messung, um ein umcodierbares Signal abzuleiten, mit einerseits einer Kapsel (1), die zumindest eine Flüssigkeit (4) aufweist sowie eine durch eine biegsame Membran (3) verschlossene Öffnung (2), die von dem aufzunehmenden Druck beaufschlagt wird und die in verschiedene Positionen verschoben werden kann, und andererseits mit einem der Öffnung (2) gegenübergelegenem Abschnitt, der mit Mitteln, die auf die Wirkung des Druckes hin elastisch verformbar sind, versehen ist, dadurch gekennzeichnet, daß die elastisch verformbaren Mittel (5, 6, 80, 83) zumindest eine Membran (5, 66, 80, 83) aufweisen, die luftdicht über einer zweiten Öffnung (7) angebracht ist, und dadurch, daß die Mittel (5, 6, 80, 83) für jeden Wert des aufgenommenen Druckes einen Gegendruck auf die Flüssigkeit (4) ausüben und die geeignet sind, sich schnell und eichbar zu verformen, um ein dem Druck entsprechendes Signal zu übermitteln.

2. Vorrichtung nach Anspruch 1, dadurch gekennzeichnet, daß sie einen aus einer photoelektrischen Empfänger (10)- und Sender (11)-Kombination bestehenden Detektor aufweist, deren optische Achse quer zu einer beweglichen Blende (5a, 6a) steht, die beweglich mit der elastischen Membran (5) verbunden ist, wobei die Membran (5) die zweite Öffnung (7) verschließt, die kleiner ist als die erste Öffnung (2) und die Kapsel (1) vollständig mit der Flüssigkeit (4) gefüllt ist.

3. Vorrichtung nach Anspruch 2, dadurch gekennzeichnet, daß die Blende durch ein gegebenenfalls gebogenes Ende (6a) einer elastischen Lamelle (6) gebildet ist, das einen Teil des elastisch verformbaren Mittels bildet und das mit seinem anderen Ende an einem festen Abschnitt befestigt sowie mit einem Metallstück (31) versehen ist, das gegenüber der Außenfläche der zweiten Membran (5) an der elastischen Lamelle befestigt ist.

4. Vorrichtung nach Anspruch 1, dadurch gekennzeichnet, daß sie einen Grundkörper (14, 20, 21) aufweist, der an seiner Außenseite die erste biegsame Membran (3) aufweist und der dazu geeignet ist, mehr oder weniger lang an einem bestimmten Punkt der Oberfläche eines lebenden Körpers zu verbleiben, so daß die Membran (3) einem Druck ausgesetzt ist, der sich wegen der radialen Vergrößerung aufgrund des Blutdruckes in einem Abschnitt einer Ader ergibt.

5. Vorrichtung nach Anspruch 4, dadurch gekennzeichnet, daß der Grundkörper (20, 21) mit starren Halterungsteilen, wie beispielsweise einem unelastischen Ring, verbunden ist.

6. Vorrichtung nach Anspruch 1, dadurch gekennzeichnet, daß die elastisch verformbaren Mittel durch ein codierendes Mittel (66) gebildet werden, die mechanische Größen in auswertbare elektrische Größen, wie elektrische, elektronische und analoge, umwandelt.

7. Vorrichtung nach Anspruch 1, dadurch gekennzeichnet, daß die Kapsel (1) eine dritte, an ihrem Rand befestigte, querliegende, verformbare Membran (50) aufweist, die zwei voneinander separierte Kammern (51, 52) schafft, die jeweils mit Flüssigkeit (4) gefüllt sind, deren eine (51) die erste Öffnung (2) aufweist und deren andere (52) die elastisch verformbaren Mittel aufweist.

8. Vorrichtung nach Anspruch 7, dadurch gekennzeichnet, daß die die elastisch verformbaren Mittel aufweisende Kammer (52) eine gekrümmte Wandung (52a) aufweist, die eine Form ähnlich der hat, die die dritte, querliegende Membran (50) annimmt, wenn sie eine maximale Verformung annimmt, so daß die querliegende Membran (50) an der gekrümmten Wandung (52a) anliegt, die somit für sie einen Anschlag bildet.

9. Vorrichtung nach Anspruch 8, dadurch gekennzeichnet, daß die die elastisch verformbaren Mittel aufweisende Kammer (52) eine gekrümmte Wandung in Form einer sphärischen Kalotte aufweist.

10. Vorrichtung nach Anspruch 1, dadurch gekennzeichnet, daß sie Mittel (66-72) aufweist, die einen der Messung entsprechenden Gegendruck an einer anderen Stelle als der, an der die Vorrichtung mit ihrer ersten biegsamen Membran (3) angebracht ist, erzeugt, wobei sich das letztendlich erhaltene Meßergebnis aus der algebraischen Summe des Druckes und des Gegendrukkes ergibt.

11. Vorrichtung nach Anspruch 10, dadurch gekennzeichnet, daß sie ein Gehäuse (67) mit zwei Einlässen (68, 69) aufweist, deren einer (68) der zweiten Öffnung der Kapsel (1) entspricht, deren Membran durch einen sich außerhalb der Flüssigkeit (4) der Kapsel (1) befindenden piezoelektrischen Detektor (66) gebildet wird, während der andere Einlaß (69) eine Röhre (72) aufnimmt, deren freies Ende (73) mit einer Membran (74) verschlossen ist, wobei die Flüssigkeit (4) die Röhre (72) und das Gehäuse (67) füllt, um mit der Seite des piezoelektrischen Detektors (66) in Kontakt zu stehen, die der mit der Flüssigkeit (4) der Kapsel (1) sich in Kontakt befindenden Seite gegenüberliegt, um eine Differenzmessung von Drücken zu erhalten.

12. Vorrichtung nach Anspruch 11, dadurch gekennzeichnet, daß das Gehäuse (67) von einer Öffnung durchstoßen ist, die durch eine biegsame Membran (75) verschlossen ist.

13. Vorrichtung nach Anspruch 1, dadurch gekennzeichnet, daß das Ende (1a) der Kapsel (1) eine Einfassung (8) bildet und die zweite Membran (80) aufnimmt, die mit einem Ansatzstück (81) verbunden ist und eine verschiebliche Blende (82) hat, die gegenüber von Queröffnungen (1b, 1c) für eine optische Detektoreinrichtung (10, 11) liegt, wobei die Membran (80) und das Ansatzstück (81) den gleichen Durchmesser haben und, bei geringem Spiel, denselben wie das Innere der Einfassung.

14. Vorrichtung nach Anspruch 1, dadurch gekennzeichnet, daß das Ende (1a) der Kapsel (1) den Scheitel einer Einfassung (8) bildet und von einem Loch durchstoßen ist, durch das sich die Achse (85) eines Kolbens (84), der außerhalb der Kapsel (1) eine Blende (86) trägt, erstreckt, wobei die zweite Membran (83) in ihrem Mittelpunkt durchstoßen ist und zwischen dem hinteren Teil (1a) der Kapsel (1) und dem Kolben (84) angeordnet ist.

## Claims

1. A device for detecting a pressure, especially of a fluid, and for utilising the same as a measurement for the purpose of deriving a trancodable signal from the same, of the type comprising, on the one hand, a capsule (1) which contains at least one liquid (4) and which is provided with an aperture (2) obturated by a flexible diaphragm (3) accessible to the pressure which is to be sensed and capable of occupying different positions and, on the other hand, a part situated opposite to the aperture (2) and provided at its end with means elastically deformable under the action of said pressure, characterized in that said elastically deformable means (5, 6, 80, 83) comprise at least one diaphragm (5, 66, 80, 83) placed in sealed manner over a second aperture (7) and in that these means (5, 6, 80, 83) exert a counter-pressure on the liquid (4) for each value of the sensed pressure and are liable to be deformed in a rapid and graded manner for the purpose of transmitting information representative of this pressure.

2. A device according to claim 1, characterized in that it comprises a detector formed by an assembly of a photoelectric cell (10) and projector (11), the light axis of which extends transversely to a moving screen (5a, 6a) coupled kinematically to the elastic diaphragm (5), this diaphragm (5) obturating said second aperture (7), this latter being smaller that the former (2), the capsule (1) being wholly filled with liquid (4).

3. A device according to claim 2, characterized in that the screen is formed by a possibly bent end (6a) of an elastic strip (6) forming part of the elastically deformable means and coupled via its other end to a fixed part (30) and provided with a stud (31) applied against the outer face of the second diaphragm (5) by the elastic strip (6).

4. A device according to claim 1, characterized in that it comprises a body (14, 20, 21) which externally has the first flexible diaphragm (3) and which is adapted to be held for a greater or lesser period at a given point of the surface of a living body so that this diaphragm (3) may be exposed to the pressure resulting from the radial volumetric increase of an arterial section in which a blood pressure develops.

5. A device according to claim 4, characterized in that the body (20, 21) is associated with rigid holding members such as an inelastic wrist strap.

6. A device according to claim 1, characterized in that the elastically deformable means are formed by an element (66) for transcoding mechanical data into exploitable data such as those of electrical, electronic and analogous nature.

7. A device according to claim 1, characterized in that the capsule (1) contains a third, deformable and transversal diaphragm fixed by its periphery and which defines two isolated chambers (51 and 52) filled with liquid (4), of which the one (51) comprises the first aperture (2) and the other (52) comprises the elastically deformable means.

8. A device according to claim 7, characterized in that the chamber (52) comprising the elastically deformable means has a curved wall (52a) having a shape equivalent to that which may be assumed by the third transversal diaphragm (50) when it undergoes a maximum deformation so that this transversal diaphragm (50) may then bear on the curved wall (52a) acting as a stop for it.

9. A device according to claim 8, characterized in that the chamber (52) comprising the elastically deformable means has a curved wall (52a) in the form of a spherical cap.

10. A device according to claim 1, characterized in that it comprises means (66-72) for applying to it a counter-pressure resulting from a measurement taken at a different point from that to which the device is applied by its first flexible diaphragm (3), the measurement finally obtained resulting from the algebraic sum of the pressure and of the counter-pressure.

11. A device according to claim 10, characterized in that it includes a housing (67) comprising two entries (68 and 69), of which the one (68) corresponds to the second aperture of the capsule (1) of which the diaphragm is formed by a piezoelectric detector (66) and externally the liquid (4) of the capsule (1) whereas the other (69) receives a tube (72) the free end (73) of which is closed by a diaphragm (74), the liquid (4) filling the tube (72) and the housing (67) to be in contact with the piezoelectric detector (66) but via its face opposed to that which receives the liquid (4) from the capsule (1) to secure a differential measurement for the pressures.

12. A device according to claim 11, characterized in that the housing (67) is traversed by an aperture obturated by a flexible diaphragm (75).

13. A device according to claim 1, characterized in that the end (1a) of the capsule (1) forms the top of a collar (8) and receives the second diaphragm (80) associated with an end piece (81) carrying a screen (82) capable of being situated in alignment with transverse apertures (1b and 1c) for an optical detector assembly (10-11), the diaphragm (80) and the end piece (81) having the same cross-section, which is equal to that of the inside of the collar (8), subject to play.

14. A device according to claim 1, characterized in that the end (1a) of the capsule (1) forms the top of a collar (8) and is pierced by a hole through which extends the axle (85) of a piston (84) carrying a screen (86) external to the capsule (1), the second diaphragm (83) being pierced in its centre and being interposed between the back (1a) of the capsule (1) and the piston (84).

FIG.1

FIG.2

FIG.3

FIG.4

FIG. 5

FIG. 6

FIG. 7

FIG. 8

FIG. 9

FIG.10

FIG.11

FIG.12

FIG.13